Europäisches Patentamt

(19) European Patent Office

Office européen des brevets

(11) Publication number : 0 422 727 B1

(12) EUROPEAN PATENT SPECIFICATION

(45) Date of publication of patent specification :
28.04.93 Bulletin 93/17

(51) Int. Cl.⁵ : C07C 15/02, C07C 7/148

(21) Application number : 90202635.0

(22) Date of filing : 04.10.90

(54) Method for recovering trimethylbenzenes from mixtures which contain them.

(30) Priority : 06.10.89 IT 2193689

(43) Date of publication of application :
17.04.91 Bulletin 91/16

(45) Publication of the grant of the patent :
28.04.93 Bulletin 93/17

(84) Designated Contracting States :
AT BE CH DE DK ES FR GB GR LI LU NL SE

(56) References cited :
US-A- 3 375 290

(73) Proprietor : Enichem Anic S.r.l.
Via Ruggero Settimo 55
I-90139 Palermo (IT)
Proprietor : ENIRICERCHE S.p.A.
Corso Venezia 16
I-20121 Milan (IT)

(72) Inventor : Messina, Giuseppe
Via Perpignan 27
I-07041 Alghero, Sassari (IT)
Inventor : Moretti, Mario Domenico
Via Oriani 5
I-07100 , Sassari (IT)
Inventor : Orlandoni, Anna Maria
Via Gramsci 9
I-07041 Alghero, Sassari (IT)
Inventor : Sanna, Salvatore Roberto
Via Biasi 1,
I-07037 Sorso, Sassari (IT)

(74) Representative : Roggero, Sergio et al
Ing. Barzanò & Zanardo Milano S.p.A. Via
Borgonuovo 10
I-20121 Milano (IT)

**Description**

The present invention relates to a method for recovering trimethylbenzenes from mixtures in which said compounds are contained together with ethyl-toluenes and possibly also butyl-benzenes.

Trimethylbenzenes and in particular mesitylen (1,3,5-trimethylbenzene) and pseudocumene (1,2,4,-trimethylbenzene) are very valuable chemicals, which find an use in several applications. In particular, for instance, pseudocumene constitutes the starting material for the synthesis of trimellitic anhydride, a compound Which constitutes the raw material for the synthesis of plasticizers, and is used as an intermediate in the industry of dyes and resins; whilst mesitylen is used as an intermediate for the synthesis of fine chemicals such as, e.g., trimesic acid, antioxidants, and so forth.

Trimethylbenzenes as contained in large amounts in a largely available stream such as the aromatic $C_9$ fraction from reforming.

Unfortunately, owing to the simultaneous presence in this fraction of the ethyl-toluenes, and in particular of the ortho-isomer thereof, having boiling points which are very close to those of trimethylbenzenes, the separation of pseudocumene and mesitylen from said mixture is particularly difficult.

Such an operation is even more difficult when the fraction used as the starting material, besides containing ethyl-toluenes, also contains butyl-benzenes (see Table I, in which the boiling points under atmospheric pressure of such compounds are reported).

Even if they are present in considerably smaller amounts than ethyl-toluenes, butyl-benzenes are particularly undesirable as during the successive oxidative transformations of pseudocumene and mesitylen they originate -- in a much easier way than ethyl-toluene do -- impurities of phenolic character which act as inhibitors of the oxidation reaction.

Table I

| Compound | Boiling point ($^\circ$C) |
|---|---|
| * Isopropylbenzene (cumene) | 152.4 |
| * n-Propylbenzene | 159.2 |
| * 3-Ethyltoluene | 161.3 |
| * 4-Ethyltoluene | 162.0 |
| * 1,3,5-trimethylbenzene (mesitylen) | 164.7 |
| * 2-Ethyltoluene | 165.2 |
| * 1,2,4-trimethylbenzene (pseudocumene) | 169.4 |
| * t-Butylbenzene | 169.5 |
| * Isobutylbenzene | 172.8 |
| * sec.-Butylbenzene | 173.6 |
| * 1,2,3-trimethylbenzene (hemimellitene) | 176.1 |
| * n-Butylbenzene | 183.6 |

At present, pseudocumene is recovered by means of a process of superfractionation, but the so obtained product does not anyway show a high enough purity degree. On the other hand, mesitylen is preferably prepared by synthesis, generally by means of the isomerization of pseudocumene under particular conditions.

2

The method according to the present invention makes it possible, on the contrary, the conventional distillation techniques to be used. These are techniques requiring low industrial costs, for recovering mesitylen and pseudocumene from mixtures which contain such products together with ethyltoluenes and possibly also butylbenzenes.

In particular, the method according to the present invention makes it possible mesitylen and pseudocumene contained in aromatic $C_9$ mixtures from reforming to be easily and cheaply separated in a form substantially free from such impurities as those listed hereinabove.

In fact, the present Applicant found that when a mixture comprising trimethylbenzenes and ethyl-toluenes, such as an aromatic $C_9$ stream from reforming, is reacted with benzene in the presence of a suitable heterogeneous acidic catalyst under transalkylation conditions, ethyl-toluenes can be preferentially or selectively converted into light products such as toluene and ethyl-benzene, which can be easily separated by distillation from trimethyl-benzenes.

Therefore, the preferential or selective transalkylation of ethyl-toluenes with benzene according to the method of the present invention involves the additional advantage that the simultaneous production is obtained, besides toluene, of ethylbenzene, a very interesting chemical at the industrial level, as an intermediate in the synthesis of styrene. Furthermore, under these same conditions any possibly present butyl-benzenes undergo a drastic reduction in amount.

Heterogeneous acidic catalysts suitable for the purpose of the present invention are, in general, the natural silico-aluminates in the acidic form, as well as the porous crystalline catalysts in their protonic form, having pore diameters comprised within the range of from 5 to 6.5 Å.

Silico-aluminates of natural origin which can be advantageously used are, e.g., clays (montmorillonite, bentonite, halloysite, kaolin, and so forth) in their exchanged form, the so said "cation-deprived acidic earths", such as, e.g., the commercial product known on the market under the trade name Filtrol[(R)], whilst suitable synthetic, amorphous silico-aluminates are, e.g., the products disclosed in EP-A-340 868.

Suitable porous crystalline catalysts are selected, on the contrary, from the group consisting of zeolites and zeolite-like materials, in their protonic form, with structures of MFI, MEL, TON, MTT or FER types.

For an indication in greater detail of the characteristics associated with each structure code, a complete enough list of the several "homotypes" corresponding to each structure and the literature references relevant to the description of the catalyst and to its preparation, reference is made to the second edition of "Atlas of Zeolite Structure Types", Editors W.M. Meier and D.H. Olson, Butterworths (1987).

When they are synthetized in their alkaline form, such catalysts are easily converted into their protonic form by means of well-known reaction of ion exchange, such as, e.g., by means of the intermediate formation of their ammonium form by means of the exchange with ammonium cations and calcination of the so obtained ammonium form, or by treatment with an acid, such as, e.g., hydrochloric acid.

According to a preferred aspect of the present invention, the transalkylation is carried out in the presence of a porous crystalline catalyst having one of the above structures, as in that way the selective transalkylation of ethyl-toluenes is obtained, with 2-ethyl-toluene being converted to a substantially complete extent and 3- and 4-ethyl-toluenes being converted to a practically complete extent; the amounts of any possibly present butylbenzenes being drastically decreased, and an only marginal transformation of mesitylen and pseudocumene being experienced.

Although they lead to the nearly total disappearance of the ethyl-toluenes, and therefore make it possible the separation of mesitylen and pseudocumene to be carried out in an equally satisfactory way, the natural, pore-free silico-aluminates also cause a not negligible conversion of trimethylbenzenes in that the transalkylation takes place only preferentially, and not selectively to the damage of ethyl-toluenes.

According to a more preferred aspect of the present invention, the transalkylation is carried out by using a porous, crystalline catalyst in its protonic form, with a structure of MFI type, such as, e.g., the catalysts based on silicon, titanium and aluminum oxides as disclosed in EP-A-266257, ZSM-5 (see U.S. patent N. 3,702,886), AMS-1B (see U.S. patent N. 4,269,813), Boralite-C (see U.K. patent N. 2,024,7790), encilite (see EP-A-160,136), TSZ-III (see EP-A-170,751), USI-108 (see U.S. patent 4,423,020), ZKQ-1B (see EP-A-148,038), and so forth.

The reaction of transalkylation is generally carried out by reacting the mixture containing the trimethylbenzenes and ethyl-toluenes with benzene in a molar ratio of benzene to $C_9$ comprised within the range of from 0.5 to 20. However, a molar ratio of benzene to $C_9$ higher than, or equal to, 1, is preferably used, and still more preferably, also on considering the ease with which unreacted benzene can be recovered by simple distillation, a molar ratio of benzene to $C_9$ higher than, or equal to, 3, is used.

The transalkylation is carried out at a temperature typically comprised within the range of from 200°C to 600°C, according to the type of catalyst used. In fact, in particular, when the catalyst is an acidic earth, the transalkylation will be preferably carried out at temperatures comprised within the range of from 200°C to 350°C, and still more preferably at temperatures comprised within the range of from 240°C to 280°C. On the contrary, when catalysts of zeolite type, active up to much higher temperatures, are used, the reaction is preferably carried out at temperatures higher than 250°C and still more preferably at temperatures comprised within the range of from 300°C to 450°C.

When the reaction of transalkylation is carried out at high temperatures, in order to avoid or minimize the coking reactions, resulting in a catalyst poisoning, the use is preferred, according to the methodologies known in the art, of a catalyst of zeolitic type containing hydrogenated metals, with the reaction being preferably carried out in the presence of a hydrogen flow.

However, the use of catalysts of zeolitic type, such as those as indicated hereinabove, suitably treated according to methods well-known in the present field in order to increase their activity, is always possible, and sometimes recommended.

The reaction of transalkylation can be carried out at atmospheric pressure, but is preferably carried out under increased pressure. In particular, pressures of up to 100 atmospheres can be advantageously used.

The reaction is carried out in practice by causing the mixture, to which benzene is previously added, to flow on a fixed bed of the selected catalyst, under selected temperature and pressure conditions.

The hourly space velocity (LHSV) can be comprised within the range of from 0.05 to 25, and preferably of from 0.5 to 10.

The catalyst, in particular in the case of catalyst of zeolitic type, is generally used in mixture with an inert binding/diluent agent and is typically prepared in the form of pellets or microspheres, as well-known in the art.

Once that it is collected, the effluent from the transalkylation area can be separated into fractions or individual products by simple distillation, or by means of other separation methods known in the art.

The following examples are reported for the purpose of better illustrating the process of the present invention as regards some of its representative aspects.

General Method

To an aromatic $C_9$ stream from reforming a determined amount of benzene is added, with a mixture being obtained which has the composition as shown in the first column on the left of the table relevant to each example. Such a mixture is made flow on a stationary bed of the indicated catalyst under such conditions of pressure, temperature and hourly space velocity as specifically indicated.

The analysis of the effluent is reported in the column to the right of each table.

The treatment of separation of mesitylen and pseudocumene by distillation is carried out according to the conventional methods, which therefore are not expressly specified herein. At the end of each example, in order to clarify the obtained results, the conversions of trimethylbenzenes and ethyltoluenes are reported.

Example 1

 * Ratio of $C_9$ mixture/benzene = 1/9
 * Catalyst: Filtrol[R]
 * Temperature = 250°C
 * Pressure = 30 kg/cm$^2$ (30,4 bar)
 * LHSV (hourly space velocity) = 2

|  | Reaction stream | Effluent |
|---|---|---|
| Light fractions | 0.03 | 0.10 |
| Benzene | 91.30 | 88.29 |

| | | |
|---|---|---|
| 3- and 4-Ethyltoluene | 2.58 | 0.39 |
| Mesitylen | 1.05 | 0.86 |
| 2-Ethyl-toluene | 0.70 | - |
| Pseudocumene | 3.16 | 1.75 |
| Hemimellitene | 0.40 | 0.20 |
| Butylbenzenes | 0.27 | 0.13 |
| Others | 0.13 | 0.20 |
| * Trimethylbenzenes | 4.61 | 2.85 |
| * Ethyltoluenes | 3.38 | 0.39 |

Conversion of Trimethyl-benzenes _____ 38%

Mesitylen 18%

Pseudocumene 45%

Hemimellitene 50%

Conversion of Ethyl-toluenes _____ 88%

3-and 4-Ethyltoluene 85%

2-Ethyltoluene 100%

Conversion of Butyl benzenes _____ 52%

Example 2

* Ratio of C$_9$ mixture/benzene = 1/9
* Catalyst: Filtrol$^{(R)}$
* Temperature = 260°C
* Pressure = 30 kg/cm$^2$
* LHSV = 5.3

| | Reaction stream | Effluent |
|---|---|---|
| Light fractions | 0.03 | 0.10 |
| Benzene | 91.30 | 87.75 |
| Toluene | - | 3.70 |
| Ethylbenzene | - | 2.67 |
| m- and p-Xylene | - | 1.31 |
| Cumene | 0.03 | 0.07 |
| o-Xylene | - | 0.30 |
| n-Propylbenzene | 0.35 | 0.48 |

| | | |
|---|---|---|
| 3- and 4-Ethyltoluene | 2.58 | 0.34 |
| Mesitylen | 1.05 | 0.94 |
| 2-Ethyl-toluene | 0.70 | - |
| Pseudocumene | 3.16 | 1.79 |
| Hemimellitene | 0.40 | 0.16 |
| Butylbenzenes | 0.27 | 0.10 |
| Others | 0.13 | 0.20 |
| * Trimethylbenzenes | 4.61 | 2.89 |
| * Ethyltoluenes | 3.38 | 0.34 |

Conversion of Trimethyl-
benzenes _____37%

Mesitylen        10%

Pseudocumene     43%

Hemimellitene    60%

Conversion of Ethyl-
toluenes _____90%

3- and 4-Ethyltoluene   87%

2-Ethyltoluene          100%

Conversion of Butyl
benzenes _____63%

Example 3

* Ratio of C$_9$ mixture/benzene = 1/9
* Catalyst: Filtrol$^{(R)}$
* Temperature = 260°C
* Pressure = 30 kg/cm$^2$
* LHSV = 7

| | Reaction stream | Effluent |
|---|---|---|
| Light fractions | 0.03 | 0.09 |
| Benzene | 91.30 | 88.31 |
| Toluene | - | 2.87 |
| Ethylbenzene | - | 2.40 |
| m- and p-Xylene | - | 0.81 |
| Cumene | 0.03 | 0.10 |
| o-Xylene | - | 0.31 |
| n-Propylbenzene | 0.35 | 0.48 |

6

| | | |
|---|---|---|
| 3- and 4-Ethyltoluene | 2.58 | 0.60 |
| Mesitylen | 1.05 | 1.05 |
| 2-Ethyl-toluene | 0.70 | - |
| Pseudocumene | 3.16 | 2.06 |
| Hemimellitene | 0.40 | 0.22 |
| Butylbenzenes | 0.27 | 0.15 |
| Others | 0.13 | 0.55 |
| * Trimethylbenzenes | 4.61 | 3.33 |
| * Ethyltoluenes | 3.38 | 0.60 |

| | | |
|---|---|---|
| Conversion of Trimethyl-benzenes _____ 28% | Conversion of Ethyl-toluenes _____ 92% |
| Mesitylen          0% | 3- and 4-Ethyltoluene   77% |
| Pseudocumene       35% | 2-Ethyltoluene         100% |
| Hemimellitene      45% | Conversion of Butyl benzenes _____ 44% |

Example 4

* Ratio of C$_9$ mixture/benzene = 1/9
* Catalyst: Filtrol$^{(R)}$
* Temperature = 270°C
* Pressure = 30 kg/cm$^2$
* LHSV = 6.9

| | Reaction stream | Effluent |
|---|---|---|
| Light fractions | 0.03 | 0.16 |
| Benzene | 91.30 | 87.50 |
| Toluene | - | 3.80 |
| Ethylbenzene | - | 2.62 |
| m- and p-Xylene | - | 1.30 |
| Cumene | 0.03 | 0.13 |
| o-Xylene | - | 0.46 |
| n-Propylbenzene | 0.35 | 0.55 |

7

| | | |
|---|---|---|
| 3- and 4-Ethyltoluene | 2.58 | 0.25 |
| Mesitylen | 1.05 | 0.82 |
| 2-Ethyl-toluene | 0.70 | - |
| Pseudocumene | 3.16 | 1.61 |
| Hemimellitene | 0.40 | 0.20 |
| Butylbenzenes | 0.27 | 0.08 |
| Others | 0.13 | 0.52 |
| * Trimethylbenzenes | 4.61 | 2.63 |
| * Ethyltoluenes | 3.28 | 0.25 |

| Conversion of Trimethyl-benzenes_____43% | Conversion of Ethyl-toluenes_____92% |
|---|---|
| Mesitylen 22% | 3- and 4-Ethyltoluene 90% |
| Pseudocumene 49% | 2-Ethyltoluene 100% |
| Hemimellitene 50% | Conversion of Butyl benzenes_____70% |

Example 5

* Ratio of C$_9$ mixture /benzene = 1/9
* Catalyst: Filtrol$^{(R)}$
* Temperature = 270°C
* Pressure = 30 kg/cm$^2$
* LHSV = 9.8

| | Reaction stream | Effluent |
|---|---|---|
| Light fractions | 0.03 | 0.08 |
| Benzene | 91.30 | 88.50 |
| Toluene | - | 3.01 |
| Ethylbenzene | - | 2.14 |
| m- and p-Xylene | - | 0.83 |
| Cumene | 0.03 | 0.12 |
| o-Xylene | - | 0.38 |
| n-Propylbenzene | 0.35 | 0.48 |

| | | |
|---|---|---|
| 3- and 4-Ethyltoluene | 2.58 | 0.75 |
| Mesitylen | 1.05 | 0.98 |
| 2-Ethyl-toluene | 0.70 | 0.10 |
| Pseudocumene | 3.16 | 2.04 |
| Hemimellitene | 0.40 | 0.25 |
| Butylbenzenes | 0.27 | 0.14 |
| Others | 0.13 | 0.20 |
| * Trimethylbenzenes | 4.61 | 3.52 |
| * Ethyltoluenes | 3.28 | 0.85 |

| | | |
|---|---|---|
| Conversion of Trimethyl-benzenes _____24% | Conversion of Ethyl-toluenes _____74% | |
| Mesitylen 7% | 3- and 4-Ethyltoluene 71% | |
| Pseudocumene 35% | 2-Ethyltoluene 86% | |
| Hemimellitene 37% | Conversion of Butyl benzenes _____48% | |

### Example 6

* Ratio of $C_9$ mixture/benzene = 1/4
* Catalyst: Boralite C
* Temperature = 320°C
* Pressure = 35 kg/cm$^2$ (35,5 bar)
* LHSV = 2

| | Reaction stream | Effluent |
|---|---|---|
| Light fractions | 0.04 | 0.24 |
| Benzene | 80.46 | 75.71 |
| Toluene | - | 5.89 |
| Ethylbenzene | - | 5.10 |
| m- and p-Xylene | - | 0.90 |
| Cumene | 0.08 | 0.11 |
| o-Xylene | - | 0.55 |
| n-Propylbenzene | 0.75 | 0.72 |

| | | |
|---|---|---|
| 3- and 4-Ethyltoluene | 5.25 | 0.95 |
| Mesitylen | 2.21 | 2.41 |
| 2-Ethyl-toluene | 1.53 | 0.10 |
| Pseudocumene | 6.62 | 5.45 |
| Hemimellitene | 1.32 | 0.78 |
| Butylbenzenes | 0.89 | 0.18 |
| Others | 0.85 | 0.91 |
| * Trimethylbenzenes | 10.15 | 8.64 |
| * Ethyltoluenes | 6.78 | 1.05 |

| | | | |
|---|---|---|---|
| Conversion of Trimethyl-benzenes _____15% | | Conversion of Ethyl-toluenes _____84% | |
| Mesitylen | 18% | 3- and 4-Ethyltoluene | 93% |
| Pseudocumene | 41% | 2-Ethyltoluene | 80% |
| Hemimellitene | 60% | Conversion of Butyl benzenes _____63% | |

Example 7

* Ratio of C$_9$ mixture/benzene = 1/4
* Catalyst: Catalyst as disclosed in Example 1 of European Patent Application N.89201118.0 filed on May 1,1989
* Temperature = 350°C
* Pressure = 35 kg/cm$^2$
* LHSV = 1

| | Reaction stream | Effluent |
|---|---|---|
| Light fractions | 0.03 | 0.38 |
| Benzene | 80.84 | 77.02 |
| Toluene | - | 4.58 |
| Ethylbenzene | - | 2.81 |
| m- and p-Xylene | - | 1.78 |
| Cumene | 0.20 | 0.50 |
| o-Xylene | 0.14 | 0.76 |

| | | |
|---|---|---|
| n-Propylbenzene | 0.84 | 1.10 |
| 3- and 4-Ethyltoluene | 4.91 | 2.69 |
| Mesitylen | 2.01 | 1.85 |
| 2-Ethyl-toluene | 1.35 | 0.42 |
| Pseudocumene | 5.68 | 3.73 |
| Hemimellitene | 1.31 | 0.54 |
| Butylbenzenes | 1.38 | 0.55 |
| Others | 1.32 | 1.49 |
| * Trimethylbenzenes | 9.00 | 6.12 |
| * Ethyltoluenes | 6.26 | 3.11 |

| Conversion of Trimethyl-benzenes_____32% | Conversion of Ethyl-toluenes_____50% |
|---|---|
| Mesitylen 8% | 3-and 4-Ethyltoluene 45% |
| Pseudocumene 34% | 2-Ethyltoluene 69% |
| Hemimellitene 59% | Conversion of Butyl benzenes_____60% |

Example 8

* Ratio of C$_9$ mixture/benzene = 1/4
* Catalyst: Catalyst 31-200 based on Zeolite Y Linde$^{(R)}$ Type, by Union Carbide
* Temperature : 290°C
* Pressure = 35 kg/cm$^2$
* LHSV = 2.5

| | Reaction stream__ | Effluent |
|---|---|---|
| Light fractions | 0.023 | 0.43 |
| Benzene | 80.59 | 76.33 |
| Toluene | - | 4.60 |
| Ethylbenzene | - | 3.06 |
| m- and p-Xylene | - | 1.57 |
| Cumene | 0.20 | 0.41 |

| | | |
|---|---|---|
| o-Xylene | 0.14 | 0.98 |
| n-Propylbenzene | 0.83 | 1.12 |
| 3- and 4-Ethyltoluene | 4.86 | 2.21 |
| Mesitylen | 1.96 | 1.91 |
| 2-Ethyl-toluene | 1.32 | 0.51 |
| Pseudocumene | 5.65 | 3.52 |
| Hemimellitene | 1.40 | 0.79 |
| Butylbenzenes | 1.21 | 0.47 |
| Others | 1.81 | 1.33 |
| * Trimethylbenzenes | 9.01 | 6.22 |
| * Ethyltoluenes | 6.18 | 2.72 |

| | | | |
|---|---|---|---|
| Conversion of Trimethyl-benzenes _____ 31% | | Conversion of Ethyl-toluenes _____ 56% | |
| Mesitylen | 2% | 3- and 4-Ethyltoluene | 54% |
| Pseudocumene | 38% | 2-Ethyltoluene | 61% |
| Hemimellitene | 44% | Conversion of Butyl benzenes _____ 61% | |

## Example 9

    * Ratio of $C_9$ mixture/benzene = 1/4
    * Catalyst: Catalyst of zeolitic type containing silicon, titanium and aluminum oxides, having the following composition:
        * $SiO_2/Al_2O_3$ = 210 and
        * $SiO_2/TiO_2$ = 120,
        prepared by substantially following the procedure as disclosed in Example 1 of EP-A-266.25, using a reaction mixture having the following composition:
        * $SiO_2/Al_2O_3$ = 300,
        * $SiO_2/TiO_2$ = 20,
        * $TPA^+/SiO_2$ = 0.25 and
        * $H_2O/SiO_2$ = 40
        and crystallizing at 100°C for 5 days.
    * Temperature = 320°C
    * Pressure = 35 kg/cm$^2$
    * LHSV = 2

|  | Reaction stream | Effluent |
|---|---|---|
| Light fractions | 0.02 | 0.12 |
| Benzene | 80.68 | 79.43 |
| Toluene | - | 1.44 |
| Ethylbenzene | - | 1.14 |
| m- and p-Xylene | - | 0.50 |
| Cumene | 0.23 | 0.52 |
| o-Xylene | 0.15 | 0.14 |
| n-Propylbenzene | 0.97 | 1.16 |
| 3- and 4-Ethyltoluene | 4.88 | 3.47 |
| Mesitylen | 1.74 | 1.77 |
| 2-Ethyl-toluene | 1.19 | 0.79 |
| Pseudocumene | 5.74 | 5.31 |
| Hemimellitene | 1.10 | 0.99 |
| Butylbenzenes | 1.24 | 1.10 |
| Others | 1.83 | 2.12 |
| * Trimethylbenzenes | 8.58 | 8.07 |
| * Ethyltoluenes | 6.07 | 4.26 |

| Conversion of Trimethyl-benzenes | 6% | Conversion of Ethyl-toluenes | 30% |
|---|---|---|---|
| Mesitylen | -2% | 3- and 4-Ethyltoluene | 29% |
| Pseudocumene | 7% | 2-Ethyltoluene | 33% |
| Hemimellitene | 10% | Conversion of Butyl benzenes | 11% |

Example 10

* Ratio of C$_9$ mixture/benzene = 1/4
* Catalyst: Catalyst of zeolitic type containing silicon, titanium and aluminum oxides, having the following composition:
  * SiO$_2$/Al$_2$O$_3$ = 210 and
  * SiO$_2$/TiO$_2$ = 120,
    prepared by substantially following the procedure as disclosed in Example 1 of EP-A-266.25, using a reaction mixture having the following composition:
  * SiO$_2$/Al$_2$O$_3$ = 300,
  * SiO$_2$/TiO$_2$ = 20,
  * TPA$^+$/SiO$_2$ = 0.25 and
  * H$_2$O/SiO$_2$ = 40
    and crystallizing at 100°C for 5 days.

* Temperature = 350°C
* Pressure = 35 kg/cm$^2$
* LHSV = 1

|  | Reaction stream | Effluent |
|---|---|---|
| Light fractions | 0.02 | 0.31 |
| Benzene | 80.68 | 77.32 |
| Toluene | - | 3.88 |
| Ethylbenzene | - | 3.67 |
| m- and p-Xylene | - | 0.64 |
| Cumene | 0.23 | 0.56 |
| o-Xylene | 0.15 | 0.20 |
| n-Propylbenzene | 0.97 | 1.07 |
| 3- and 4-Ethyltoluene | 4.88 | 1.75 |
| Mesitylen | 1.74 | 1.93 |
| 2-Ethyl-toluene | 1.19 | 0.19 |
| Pseudocumene | 5.74 | 5.00 |
| Hemimellitene | 1.10 | 0.79 |
| Butylbenzenes | 1.24 | 0.58 |
| Others | 1.83 | 2.11 |
| * Trimethylbenzenes | 8.58 | 7.72 |
| * Ethyltoluenes | 6.07 | 1.94 |

Conversion of Trimethyl-benzenes_____10%

Mesitylen                -11%

Pseudocumene         13%

Hemimellitene          28%

Conversion of Ethyl-toluenes_____68%

3- and 4-Ethyltoluene   64%

2-Ethyltoluene              84%

Conversion of Butyl benzenes_____53%

Example 11

* Ratio of C$_9$ mixture/benzene = 1/4
* Catalyst: Catalyst of zeolitic type containing silicon, titanium and aluminum oxides, having the following composition:
    * SiO$_2$/Al$_2$O$_3$ = 210 and
    * SiO$_2$/TiO$_2$ = 120,
        prepared by substantially following the procedure as disclosed in Example 1 of EP-A-266.257, using a reaction mixture having the following composition:

14

* $SiO_2/Al_2O_3 = 300$,
* $SiO_2/TiO_2 = 20$,
* $TPA^+/SiO_2 = 0.25$ and
* $H_2O/SiO_2 = 40$
  and crystallizing at 100°C for 5 days.
* Temperature = 380°C
* Pressure = 35 kg/cm$^2$
* LHSV = 1

| | Reaction stream | Effluent |
|---|---|---|
| Light fractions | 0.02 | 0.49 |
| Benzene | 80.68 | 76.90 |
| Toluene | - | 5.16 |
| Ethylbenzene | - | 4.82 |
| m- and p-Xylene | - | 1.00 |
| Cumene | 0.23 | 0.40 |
| o-Xylene | 0.15 | 0.16 |
| n-Propylbenzene | 0.97 | 1.01 |
| 3- and 4-Ethyltoluene | 4.88 | 0.72 |
| Mesitylen | 1.74 | 1.97 |
| 2-Ethyl-toluene | 1.19 | 0.01 |
| Pseudocumene | 5.74 | 4.60 |
| Hemimellitene | 1.10 | 0.73 |
| Butylbenzenes | 1.24 | 0.12 |
| Others | 1.83 | 1.91 |
| * Trimethylbenzenes | 8.58 | 7.30 |
| * Ethyltoluenes | 6.07 | 0.73 |

| Conversion of Trimethylbenzenes _____ 15% | Conversion of Ethyltoluenes _____ 88% |
|---|---|
| Mesitylen -13% | 3- and 4-Ethyltoluene 85% |
| Pseudocumene 20% | 2-Ethyltoluene 99% |
| Hemimellitene 34% | Conversion of Butyl benzenes _____ 90% |

## Claims

1. Method for recovering trimethylbenzenes from mixtures in which such compounds are contained together with ethyl-toluenes, and possibly also with butyl-benzenes, which method consists of treating such a mixture with benzene in the presence of a heterogeneous acidic catalyst selected from the group consisting of the natural or synthetic pore-free alumino-silicates and porous crystalline catalysts, having pores of diameter comprised within the range of from 5 to 6.5 Å, in protonic form, under conditions of transalkylation, and of subsequently separating the trimethylbenzes by distillation.

2. Method according to claim 1, wherein benzene is used in a molar ratio of benzene/$C_9$ comprised within the range of from 0.5 to 20.

3. Method according to claim 1, wherein the catalyst is selected from the group consisting of natural and synthetic alumino-silicates in their exchanged form.

4. Method according to claim 1, wherein the catalyst is selected from the group consisting of the porous crystalline catalysts in the protonic form having structures of MFI, MEL, MTT, FER or TON type.

5. Method according to claim 4, wherein the catalyst has a structure of MFI type.

6. Method according to any of the preceding claims, wherein the mixture used as the starting material is a $C_9$ aromatic stream from reforming.

7. Method according to claim 3, wherein the reaction of transalkylation is carried out at a temperature comprised within the range of from 200°C to 350°C.

8. Method according to claim 7, wherein the translkylation is carried out at a temperature comprised within the range of from 240°C to 280°C.

9. Method according to claim 4, wherein the reaction of transalkylation is carried out at a temperature comprised within the range of from 200°C to 600°C

10. Method according to claim 9, wherein the reaction of transalkylation is carried out at a temperature comprised within the range of from 300°C to 450°C.

11. Method according to claim 1, wherein the reaction of transalkylation is carried out at atmospheric pressure or under a higher-than-atmospheric pressure.

12. Method according to claim 1, wherein the hourly space velocity is comprised within the range of from 0.05 to 25.

13. Method according to claim 12, wherein the hourly space velocity is comprised within the range of from 0.5 to 10.

**Patentansprüche**

1. Verfahren zur Gewinnung von Trimethylbenzolen aus Gemischen, worin diese Verbindung zusammen mit Ethyltoluolen und gegebenenfalls auch zusammen mit Butylbenzolen enthalten sind, welches Verfahren in der Behandlung eines solchen Gemisches mit Benzol in Anwesenheit eines heterogenen sauren Katalysators, ausgewählt aus der aus natürlichen oder synthetischen porenfreien Aluminosilikaten und porösen kristallinen Katalysatoren mit Porendurchmessern im Bereich von 5 bis 6,5 Å in protonischer Form bestehenden Gruppe, unter Bedingungen einer Transalkylierung und aus dem anschließenden Abtrennen der Trimethylbenzole durch Destillation besteht.

2. Verfahren nach Anspruch 1, worin Benzol in einem Molverhältnis von Benzol/$C_9$ im Bereich von 0,5 bis 20 verwendet wird.

3. Verfahren nach Anspruch 1, worin der Katalysator aus der aus natürlichen und synthetischen Aluminosilikaten in ihrer ausgetauschten Form bestehenden Gruppe ausgewählt wird.

4. Verfahren nach Anspruch 1, worin der Katalysator aus der aus den porösen kristallinen Katalysatoren in der protonischen Form mit Strukturen vom Typ MFI, MEL, MTT, FER oder TON bestehenden Gruppe aus-

EP 0 422 727 B1

gewählt wird.

5. Verfahren nach Anspruch 4, worin der Katalysator eine Struktur vom MFI-Typ aufweist.

6. Verfahren nach einem der vorstehenden Ansprüche, worin das als Ausgangsmaterial verwendete Gemisch ein $C_9$-Aromatenstrom aus dem Reformieren ist.

7. Verfahren nach Anspruch 3, worin die Transalkylierungsreaktion bei einer Temperatur im Bereich von 200°C bis 350°C ausgeführt wird.

8. Verfahren nach Anspruch 7, worin die Transalkylierung bei einer Temperatur im Bereich von 240°C bis 280°C ausgeführt wird.

9. Verfahren nach Anspruch 4, worin die Transalkylierungsreaktion bei einer Temperatur im Bereich von 200°C bis 600°C ausgeführt wird.

10. Verfahren nach Anspruch 9, worin die Transalkylierungsreaktion bei einer Temperatur im Bereich von 300°C bis 450°C ausgeführt wird.

11. Verfahren nach Anspruch 1, worin die Transalkylierungsreaktion bei atmosphärischem Druck oder einem überatmosphärischem Druck ausgeführt wird.

12. Verfahren nach Anspruch 1, worin die Raumgeschwindigkeit je Stunde im Bereich von 0,05 bis 25 liegt.

13. Verfahren nach Anspruch 12, worin die Raumgeschwindigkeit je Stunde im Bereich von 0,5 bis 10 liegt.

## Revendications

1. Procédé de récupération de triméthylbenzènes à partir de mélanges qui contiennent ces composés conjointement avec des éthyl-toluènes, et éventuellement avec aussi des butyl-benzènes, lequel procédé consiste à traiter un tel mélange avec du benzène, en présence d'un agent acide de catalyse en phase hétérogène, choisi dans le groupe constitué par les alumino-silicates non poreux, naturels ou synthétiques, et les catalyseurs cristallins poreux dont les pores ont un diamètre situé dans l'intervalle allant de 5 à 6,5 Å, sous forme protonique, dans des conditions de transalkylation, et à séparer ensuite les triméthylbenzènes par distillation.

2. Procédé selon la revendication 1, dans lequel on utilise le benzène en un rapport molaire benzène/$C_9$ situé dans l'intervalle allant de 0,5 à 20.

3. Procédé selon la revendication 1, dans lequel le catalyseur est choisi dans le groupe constitué par les alumino-silicates naturels ou synthétiques sous leur forme échangée.

4. Procédé selon la revendication 1, dans lequel le catalyseur est choisi dans le groupe constitué par les catalyseurs cristallins poreux, sous forme protonique, ayant des structures de type MFI, MEL, MTT, FER ou TON.

5. Procédé selon la revendication 4, dans lequel le catalyseur a une structure de type MFI.

6. Procédé selon l'une quelconque des revendications précédentes, dans lequel le mélange utilisé comme matériau de départ est un courant de composés aromatiques en $C_9$ provenant d'un reformage.

7. Procédé selon la revendication 3, dans lequel la réaction de transalkylation est effectuée à une température située dans l'intervalle allant de 200°C à 350°C.

8. Procédé selon la revendication 7, dans lequel la réaction de transalkylation est effectuée à une température située dans l'intervalle allant de 240°C à 280°C.

9. Procédé selon la revendication 4, dans lequel la réaction de transalkylation est effectuée à une température située dans l'intervalle allant de 200°C à 600°C.

**10.** Procédé selon la revendication 9, dans lequel la réaction de transalkylation est effectuée à une température située dans l'intervalle allant de 300°C à 450°C.

**11.** Procédé selon la revendication 1, dans lequel la réaction de transalkylation est effectuée sous une pression égale ou supérieure à la pression atmosphérique.

**12.** Procédé selon la revendication 1, dans lequel la vitesse spatiale horaire se situe dans l'intervalle allant de 0,05 à 25.

**13.** Procédé selon la revendication 12, dans lequel la vitesse spatiale horaire se situe dans l'intervalle allant de 0,5 à 10.